# EUROPEAN PATENT APPLICATION

(11) **EP 2 243 481 A1**
(43) Date of publication of application: **27.10.2010**
(21) Application number: 09158748.5
(22) Date of filing: 24.04.2009
(51) Int. Cl.: A61K 31/4184, A61P 25/28, A61P 25/16, A61P 25/00

(54) **Irak kinase family as novel drug target for Alzheimer**

(71) Applicant: PamGene B.V., 5211 's Hertogenbosch (NL); Vereniging voor Christelijk Hoger Onderwijs, Wetenschappelijk Onderzoek en Patiëntenzorg, 1081 HV Amsterdam (NL)
(72) Inventor: Hilhorst, Maria Helena, 6706 CP, Wageningen (NL); Hoozemans, Jeroen Joseph Maria, 1061 BK, Amsterdam (NL); van der Vies, Saskia Maria, 1185 CC, Amstelveen (NL)
(74) Representative: De Clercq, Ann G. Y.

(57) **Abstract**

The present invention relates to the use of inhibitors of the IRAK protein kinase family or a pharmaceutical composition thereof in the treatment of neurological disorders such as Alzheimer's disease. Furthermore, the present invention relates to methods and devices for the diagnosis or response prediction of neurological disorders by measuring kinase activity and studying the phosphorylation levels and profiles in samples of said patients.

SEQUENCE LISTING

<110>  PamGene B.V.
       Vereniging voor christelijk hoger onderwijs,
       wetenschappelijk onderzoek en patiëntenzorg

<120>  IRAK KINASE FAMILY AS NOVEL DRUG TARGET FOR ALZHEIMER

<130>  PAM-052-EPPRIO

<160>  28

<170>  PatentIn version 3.3

<210>  1
<211>  13
<212>  PRT
<213>  Artificial

<220>
<223>  peptide kinase substrate

<400>  1

Cys Asn Ala Thr Phe Lys Lys Thr Phe Arg His Leu Leu
1               5                   10


<210>  2
<211>  13
<212>  PRT
<213>  Artificial

<220>
<223>  peptide kinase substrate

<400>  2

Cys Tyr Ala Leu Cys Asn Arg Thr Phe Arg Lys Thr Phe
1               5                   10


<210>  3
<211>  13
<212>  PRT
<213>  Artificial

<220>
<223>  peptide kinase substrate

<400>  3

Cys Asn Arg Thr Phe Arg Lys Thr Phe Lys Met Leu Leu
1               5                   10


<210>  4
<211>  13
<212>  PRT
<213>  Artificial

<220>
<223>  peptide kinase substrate

<400>  4

Glu Phe Pro Ser Arg Gly Lys Ser Ser Ser Tyr Ser Lys
1               5                   10

<210>  5
<211>  12
<212>  PRT
<213>  Artificial

<220>
<223>  peptide kinase substrate

<400>  5

Val Ala Ser Met Met His Arg Gln Glu Thr Val Glu
1               5                   10


<210>  6
<211>  13
<212>  PRT
<213>  Artificial

<220>
<223>  peptide kinase substrate

<400>  6

Phe Lys Lys Ser Phe Lys Leu Ser Gly Phe Ser Phe Lys
1               5                   10


<210>  7
<211>  13
<212>  PRT
<213>  Artificial

<220>
<223>  peptide kinase substrate

<400>  7

Glu Pro His Val Thr Arg Arg Thr Pro Asp Tyr Phe Leu
1               5                   10


<210>  8
<211>  11
<212>  PRT
<213>  Artificial

<220>
<223>  peptide kinase substrate

<400>  8

Arg Asp Lys Tyr Lys Thr Leu Arg Gln Ile Arg
1               5                   10


<210>  9
<211>  13
<212>  PRT
<213>  Artificial

<220>
<223>  peptide kinase substrate

<400>  9

Ala Val Ile Pro Ile Asn Gly Ser Pro Arg Thr Pro Arg
1               5                   10

<210>  10
<211>  13
<212>  PRT
<213>  Artificial

<220>
<223>  peptide kinase substrate

<400>  10

Asp Ala Glu Asn Arg Leu Gln Thr Met Lys Glu Glu Leu
1               5                   10


<210>  11
<211>  13
<212>  PRT
<213>  Artificial

<220>
<223>  peptide kinase substrate

<400>  11

Glu Arg Ser Lys Thr Val Thr Ser Phe Tyr Asn Gln Ser
1               5                   10


<210>  12
<211>  13
<212>  PRT
<213>  Artificial

<220>
<223>  peptide kinase substrate

<400>  12

Phe Ala Lys Thr Phe Val Gly Thr Pro Tyr Tyr Met Ser
1               5                   10


<210>  13
<211>  13
<212>  PRT
<213>  Artificial

<220>
<223>  peptide kinase substrate

<400>  13

Phe Met Arg Leu Arg Arg Leu Ser Thr Lys Tyr Arg Thr
1               5                   10


<210>  14
<211>  13
<212>  PRT
<213>  Artificial

<220>
<223>  peptide kinase substrate

<400>  14

Gly Gly Pro Thr Thr Pro Leu Ser Pro Thr Arg Leu Ser

1               5                   10


<210>  15
<211>  13
<212>  PRT
<213>  Artificial

<220>
<223>  peptide kinase substrate

<400>  15

Gly Ser Pro Asn Arg Ala Tyr Thr His Gln Val Val Thr
1               5                   10


<210>  16
<211>  13
<212>  PRT
<213>  Artificial

<220>
<223>  peptide kinase substrate

<400>  16

Gly Ser Pro Ser Lys Ser Pro Ser Lys Lys Lys Lys Lys
1               5                   10


<210>  17
<211>  13
<212>  PRT
<213>  Artificial

<220>
<223>  peptide kinase substrate

<400>  17

His Phe Asp Glu Arg Asp Lys Thr Ser Arg Asn Met Arg
1               5                   10


<210>  18
<211>  13
<212>  PRT
<213>  Artificial

<220>
<223>  peptide kinase substrate

<400>  18

Ile Ala Lys Arg Arg Arg Leu Ser Ser Leu Arg Ala Ser
1               5                   10


<210>  19
<211>  13
<212>  PRT
<213>  Artificial

<220>
<223>  peptide kinase substrate

<400>  19

Lys Ile Pro Lys Arg Pro Gly Ser Val His Arg Thr Pro
1               5                   10

<210>  20
<211>  13
<212>  PRT
<213>  Artificial

<220>
<223>  peptide kinase substrate

<400>  20

Lys Lys Lys Phe Arg Thr Pro Ser Phe Leu Lys Lys Ser
1               5                   10

<210>  21
<211>  13
<212>  PRT
<213>  Artificial

<220>
<223>  peptide kinase substrate

<400>  21

Lys Lys Lys Lys Lys Arg Phe Ser Phe Lys Lys Ser Phe
1               5                   10

<210>  22
<211>  13
<212>  PRT
<213>  Artificial

<220>
<223>  peptide kinase substrate

<400>  22

Lys Lys Asn Gly Arg Ile Leu Thr Leu Pro Arg Ser Asn
1               5                   10

<210>  23
<211>  13
<212>  PRT
<213>  Artificial

<220>
<223>  peptide kinase substrate

<400>  23

Pro Gly Lys Ala Arg Lys Lys Ser Ser Cys Gln Leu Leu
1               5                   10

<210>  24
<211>  13
<212>  PRT
<213>  Artificial

<220>
<223>  peptide kinase substrate

<400>  24

Pro Pro Arg Pro Arg Thr Pro Gly Arg Pro Leu Ser Ser
1               5                   10


<210>  25
<211>  13
<212>  PRT
<213>  Artificial

<220>
<223>  peptide kinase substrate

<400>  25

Gln Ser Arg Pro Arg Ser Cys Thr Trp Pro Leu Gln Arg
1               5                   10


<210>  26
<211>  13
<212>  PRT
<213>  Artificial

<220>
<223>  peptide kinase substrate

<400>  26

Arg Phe Ala Arg Lys Gly Ser Leu Arg Gln Lys Asn Val
1               5                   10


<210>  27
<211>  13
<212>  PRT
<213>  Artificial

<220>
<223>  peptide kinase substrate

<400>  27

Ser Gln Arg Gln Arg Ser Thr Ser Thr Pro Asn Val His
1               5                   10


<210>  28
<211>  13
<212>  PRT
<213>  Artificial

<220>
<223>  peptide kinase substrate

<400>  28

Val Ala Asp Gln Thr Pro Thr Pro Thr Arg Phe Leu Lys
1               5                   10

## Description

### FIELD OF THE INVENTION

The present invention relates to the use of inhibitors of the IRAK protein kinase family or a pharmaceutical composition thereof in the treatment of neurological disorders such as Alzheimer's disease. Furthermore, the present invention relates to methods and devices for the diagnosis or drug response prediction of neurological disorders by measuring kinase activity and studying the phosphorylation levels and profiles in samples of said patients.

### BACKGROUND OF THE INVENTION

Interleukin-1 plays an important role in inflammation, acting locally and systemically to induce other proinflammatory cytokines, chemotactic factors, adhesion molecules, acute phase proteins, and fever. Animals which lack expression of IL-1 or IL-1 receptor have reduced inflammatory responses. Cellular responses to IL-1 are mediated by a cascade of intracellular signalling events including activation of the stress-activated MAP kinases, c-Jun N-terminal kinase (JNK) and p38, as well as transcription factors NF-κB.

An important role in the initiation of the IL-1 signalling pathway is played by kinases of the IRAK family. IL-1 receptor-associated kinases (IRAKs) are important mediators in the signal transduction involved in host defence mechanisms, either by the recognition of pathogens or as receptors for proinflammatory cytokines. They play a crucial role in the switch from innate to adaptive immunity in mammals, and the signalling cascades initiated by these receptors are implicated in a number of human diseases.

IRAK proteins have also been shown to play an important role in transducing signals other than those originating from IL-1 receptors, including signals triggered by activation of IL-18 receptors and lipopolysaccharide, CD14 receptors or Toll-like receptors (TLRs).

The identification of compounds that modulate the function of IRAK proteins represents an attractive approach to the development of therapeutic agents for the treatment of inflammatory, cell proliferative and immune-related conditions and diseases associated with IRAK-mediated signal transduction.

The present invention aims at providing new uses of compounds inhibiting kinases of the IRAK protein kinase family. The present invention also aims to provide methods and devices for diagnosing neurological disorders.

### SUMMARY OF THE INVENTION

The present invention provides the new uses of compounds inhibiting kinases of the IRAK protein kinase family and more specifically the use of these compounds for the treatment of neurological disorders such as Alzheimer's disease. Furthermore, the present invention relates to methods and devices for the diagnosis and/or response prediction of neurological disorders by measuring kinase activity and studying the phosphorylation levels and profiles in samples of said patients.

The present invention therefore relates to drugs acting against protein kinases of the IRAK protein kinase family, preferably inhibitors of the IRAK protein kinase family, for use in the treatment of neurological disorders such as Alzheimer's disease, Huntington's disease, Parkinson's disease, Creutzfeldt-Jakob disease, other prion diseases and multiple sclerosis.

In a preferred embodiment of the present invention said neurological disorder is Alzheimer's disease.

In yet another preferred embodiment of the present invention said inhibitors of the IRAK protein kinase family are IRAK-4 or IRAK-1 protein kinase inhibitors.

The present invention therefore provides a method for diagnosing neurological disorders such as Alzheimer's disease, Huntington's disease, Parkinson's disease, Creutzfeldt-Jakob disease, other prion diseases and multiple sclerosis, in a subject. In a preferred embodiment of the present invention, the method comprises the steps of:
(a) measuring in a sample, obtained from said subject, a level of IRAK protein kinase(s), a level of IRAK protein kinase phosphorylation, a level of IRAK protein kinase activity and/or a level, phosphorylation level or kinase activity level of IRAK protein kinase pathway related proteins; and
(b) determining from said level(s) the occurrence of specific neurological disorders, thereby diagnosing neurological disorders in said subject.

The present invention therefore relates to a method for predicting the response of a subject, diagnosed with a neurological disorder, on IRAK protein kinase inhibitor(s) or pharmaceutical composition(s) comprising the same comprising the steps of:
(a) measuring in a sample, obtained from said subject, a level of IRAK protein kinase, a level of IRAK protein kinase phosphorylation, a level of IRAK protein kinase activity and/or a level, phosphorylation level or kinase activity level of IRAK protein kinase pathway related proteins; and,
(b) comparing the level(s) or profile(s) determined in step (a) with control value(s) or control profile(s) from which the clinical outcome of the treatment with said IRAK protein kinase inhibitor(s) or said pharmaceutical composition(s) comprising the same is a priori known.

These and further aspects and embodiments are described in the following sections and in the claims.

### BRIEF DESCRIPTION OF FIGURES

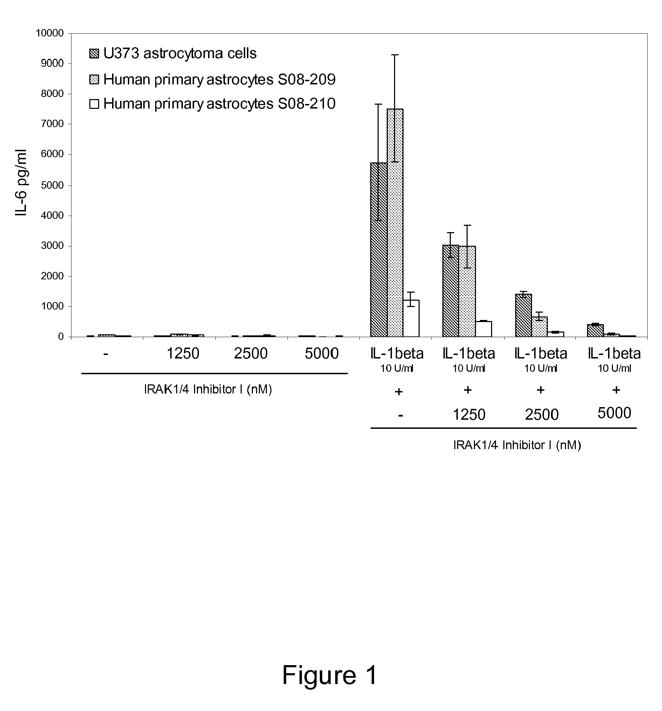

**Figure 1** provides, as depicted in the examples, a graphical representation where IL-1β induced IL-6 secretion by U373 astrocytoma cells and human primary astrocytes is reduced by IRAK1/4 inhibitor.

### DETAILED DESCRIPTION OF THE INVENTION

Before the present method and devices used in the invention are described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

All references cited in the present application are hereby incorporated by reference.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

The present invention provides the new uses of compounds inhibiting kinases of the IRAK protein kinase family and more specifically the use of these compounds for the treatment of neurological disorders such as Alzheimer's disease. Furthermore, the present invention relates to methods and devices for the diagnosis or response prediction of neurological disorders by measuring kinase activity and studying the phosphorylation levels and profiles in samples of said patients.

The inventors have surprisingly found a clear difference between the kinase activity of Cerebral Spinal Fluid (CSF) and/or tissue samples from patients suffering from Alzheimer's disease and/or other neurodegenerative disorders and the kinase activity of tissue samples from non-demented (healthy) patients. The differential kinase activity was associated to the activity of protein kinases of the IRAK protein kinase family. Consequently, the IRAK protein kinase family plays a crucial role in the development of neurological disorders such as Alzheimer's disease and protein kinases of the IRAK protein kinase family are therefore valuable drug targets in the treatment of neurological disorders such as Alzheimer's disease. A person skilled in the art will confirm that as a consequence of the close relationship between Alzheimer's disease and other neurological disorders such as Huntington's disease, Parkinson's disease, Creutzfeldt-Jakob disease, other prion diseases and multiple sclerosis, the protein kinases of the IRAK protein kinase family, and preferably IRAK-4 and/or IRAK-1, as drug targets for the treatment of Alzheimer's disease, will also be valuable drug targets for the treatment of other neurological disorders such as Huntington's disease, Parkinson's disease, Creutzfeldt-Jakob disease and other prion diseases.

The present invention therefore relates to drugs acting against protein kinases of the IRAK protein kinase family, and preferably inhibitors of the IRAK protein kinase family, for use in the treatment of neurological disorders such as Alzheimer's disease, Huntington's disease, Parkinson's disease, Creutzfeldt-Jakob disease, other prion diseases and multiple sclerosis.

In yet another embodiment, the present invention relates to inhibitors of the IRAK protein kinase family, for use in the development of a medicament for the treatment of neurological disorders such as Alzheimer's disease, Huntington's disease, Parkinson's disease, Creutzfeldt-Jakob disease, other prion diseases and multiple sclerosis.

In a preferred embodiment of the present invention said neurological disorder is Alzheimer's disease.

In yet another preferred embodiment of the present invention said inhibitors of the IRAK protein kinase family are IRAK-4 or IRAK-1 protein kinase inhibitors.

The IRAK protein kinase family consists of four related kinases involved in intracellular signalling of IL-1R and TLRs. The IRAK protein kinase family comprises IRAK-1, IRAK-2, IRAK-3 (or IRAK-M) and IRAK-4.

In yet another preferred embodiment of the present invention said IRAK protein kinase inhibitors are selected from the group consisting of small molecules, aptamers and/or genetic interferers directed toward the IRAK protein kinase family.

As used herein, inhibitors of the IRAK protein kinase family refer to compounds which inhibit the function of IRAK protein kinases and more preferably compounds which inhibit the function of IRAK-4 and/or IRAK-1. Several inhibitors of the IRAK protein kinase family have been described in the international patent applications WO 2003/030902, WO 2004/041285 and WO 2008/030579. These cited references are hereby incorporated by reference. Several inhibitors of the IRAK protein kinase family, and more specifically inhibitors of IRAK-4, have also been described in 3 publications of Buckley et al. (IRAK-4 inhibitors. Part 1: a series of amides. In Bioorganic & medicinal chemistry letters 2008, 18(11):3211-3214; IRAK-4 inhibitors. Part II: a structure-based assessment of imidazo[1,2-a]pyridine binding. In Bioorganic & medicinal chemistry letters 2008, 18(11):3291-3295; IRAK-4 inhibitors. Part III: a series of imidazo[1,2-a]pyridines. In Bioorganic & medicinal chemistry letters 2008, 18(11):3656-3660). These cited references are hereby incorporated by reference. Other known inhibitors of the IRAK protein kinase family, and more specifically inhibitors of IRAK-4 and/or IRAK-1, include, but are not limited to, R06245, R00884, N-acyl 2-aminobenzimidazoles 1-(2-(4-Morpholinyl)ethyl)-2-(3-nitrobenzoylamino)benzimidazole and/or N-(2-Morpholinylethyl)-2-(3-nitrobenzoylamido)-benzimidazole.

Aptamers, preferably high-affinity aptamers, are oligonucleic acid or peptide molecules that bind a specific target molecule. Aptamers are usually created by selecting them from a large random sequence pool. Aptamers are known to be used for both basic research and clinical purposes as macromolecular drugs. Aptamers can be generated using a process called SELEX. Said aptamers are preferably directed toward the IRAK protein kinase family, thereby inhibiting the function of proteins of the IRAK protein kinase family.

As used in the present invention, the term "genetic interferers" refer to molecules capable of mediating RNA interference. RNA interference refers to the process of sequence-specific post-transcriptional gene silencing. This process helps to control which genes are active and how active they are within living cells. More specifically two types of small RNA molecules are used in RNA interference. MicroRNA (miRNA) and small interfering RNA (siRNA) are the direct products of genes, and can bind to specific other RNAs thereby either increasing or decreasing their activity. For example by preventing a messenger RNA from producing a protein the activity can be decreased. Several publications (Zamore et al., 2000, Cell, 101, 25-33; Fire et al., 1998, Nature, 391, 806; Hamilton et al., 1999, Science, 286, 950-951; Lin et al., 1999, Nature, 402, 128-129; Sharp, 1999, Genes & Dev., 13:139-141; and Strauss, 1999, Science, 286, 886) relate to RNA interference. RNA interference is commonly used for gene knockdown, causing a decrease in the expression of a targeted gene, but the use of genetic interferers has also been exploited in therapy and biotechnology

In a further embodiment the present invention relates to a pharmaceutical composition comprising inhibitors of the IRAK protein kinase family, and preferably IRAK-4 or IRAK-1 protein kinase inhibitors, for use in the treatment of neurological disorders such as Alzheimer's disease, Huntington's disease, Parkinson's disease, Creutzfeldt-Jakob disease, other prion diseases and multiple sclerosis and preferably Alzheimer's disease.

More preferably said pharmaceutical composition according to the present invention comprises inhibitors of the IRAK protein kinase family selected from the group consisting of small molecules, aptamers and/or genetic interferers directed toward the IRAK protein kinase family.

In yet another embodiment, the present invention relates to a pharmaceutical composition comprising inhibitors of the IRAK protein kinase family, for use in the development of a medicament for the treatment of neurological disorders such as Alzheimer's disease, Huntington's disease, Parkinson's disease, Creutzfeldt-Jakob disease, other prion diseases and multiple sclerosis.

The present invention further provides methods and devices for diagnosing neurological disorders based on the measurement of the kinase activity of a sample. Preferably, in one embodiment of the present invention, methods are provided wherein the kinase activity is protein kinase activity and more preferably IRAK protein kinase activity.

For purposes of the present invention, and as used herein the terms "enzyme activity", "kinase activity" or "protein kinase activity" refer to the formation of reaction product(s) by a certain amount of enzyme, kinase or protein kinase acting on a substrate during the course of the assay.

Protein kinase activity is referred to as the activity of one or more protein kinases. A protein kinase is a generic name for all enzymes that transfer an activated phosphate group to a protein. About three to four percent of the human genome contains transcription information for the formation of protein kinases. Currently, there are about 518 known different protein kinases. However, because three to four percent of the human genome is a code for the formation of protein kinases, there may be many more separate kinases in the human body.

A protein kinase is a kinase enzyme that modifies other proteins by covalently coupling phosphate groups to them. This process or activity is also referred to as phosphorylation. Phosphorylation can therefore be regarded as the process of the addition of a phosphate group to a substrate. Phosphorylation usually results in a functional change of the substrate by changing enzyme activity, cellular location, or association with other proteins. Up to 30% of all proteins may be modified by kinase activity, and kinases are known to regulate the majority of cellular pathways, especially those involved in signal transduction, the transmission of signals within the cell. The chemical activity of a kinase involves removing a phosphate group from ATP or GTP and covalently attaching it to amino acids such as serine, threonine, tyrosine, histidine, aspartic acid and/or glutamic acid that have a free hydroxyl group. Most known kinases act on both serine and threonine, others act on tyrosine, and a number act on all serine, threonine and tyrosine. The protein kinase activity monitored with the method of the present invention is preferably directed to protein kinases acting towards serine, threonine and/or tyrosine, preferably acting on both serine and threonine, on tyrosine or on serine, threonine and tyrosine. More preferably the method of the present invention is preferably directed to protein kinases acting towards serine and threonine amino acid residues.

Protein kinases are distinguished by their ability to phosphorylate substrates on discrete sequences. These sequences have been determined by sequencing the amino acids around the phosphorylation sites and are usually distinct for each protein kinase. The recognition sequence on each substrate is for a specific kinase catalyst.

Because protein kinases have profound effects on a cell, their activity is highly regulated. Kinases are turned on or off by for instance phosphorylation, by binding of activator proteins or inhibitor proteins, or small molecules, or by controlling their location in the cell relative to their substrates. Deregulated kinase activity is a frequent cause of disease, such as for instance in cancer, where kinases regulate many aspects that control cell growth, movement and death. Therefore monitoring the protein kinase activity in tissues can be of great importance and a large amount of information can be obtained when comparing the kinase activity of different tissue samples.

For purposes of the present invention, and as used herein the term "IRAK protein kinase activity" refers to the specific protein kinases of the IRAK protein kinase family that interact with proteins involved in cell signalling including NF-kappa B as well as mitogen-activated protein (MAP) kinase pathways. The IRAK protein kinase family consists of four related kinases involved in intracellular signalling of IL-1R, IL-18R, CD14, and TLRs. The IRAK protein kinase family comprises IRAK-1, IRAK-2, IRAK-3 (or IRAK-M) and IRAK-4.

As described in the present invention, the inventors have surprisingly found that a diagnosis for neurological disorders can be determined on the basis of the measurement of the kinase activity of a sample, and preferably the IRAK kinase activity. As used in the present invention the term "diagnosis", "diagnose" of "diagnosing" refers to the process of identifying a medical condition or disease, and in the present case neurological disorders and preferably Alzheimer's disease, from the results of the methods according to the present invention. The conclusion reached through this diagnostical process is called a diagnosis.

The measurement of the kinase activity is performed by contacting a sample with one or more substrates, preferably protein kinase substrates, thereby generating a phosphorylation profile.

Said protein kinase substrates as used herein, are preferably peptides, proteins or peptide mimetics. The protein kinase substrates each comprise one or more phosphorylation sites that can be phosphorylated by the protein kinases present in the sample. Therefore, during the measurement method the kinase enzymes actively present in the sample will phosphorylate one or more of the phosphorylation sites on one or more protein kinase substrates. The inventors have observed essential differences between kinase activity of samples obtained from patients suffering from Alzheimer's disease and the kinase activity of normal samples. Consequently, the inventors have observed that the kinases present in a sample obtained from patients suffering from Alzheimer's disease will phosphorylate different protein kinase substrates compared to a normal tissue sample. This difference in kinase activity has been observed especially for IRAK protein kinase activity and more particularly for IRAK-1 and/or IRAK-4 protein kinase activity.

The present invention therefore provides a method for diagnosing neurological disorders such as Alzheimer's disease, Huntington's disease, Parkinson's disease, Creutzfeldt-Jakob disease, other prion diseases and multiple sclerosis, in a subject. In a preferred embodiment of the present invention, the method comprises the steps of:
(a) measuring in a sample, obtained from said subject, a level of IRAK protein kinase(s), a level of IRAK protein kinase phosphorylation, a level of IRAK protein kinase activity and/or a level, phosphorylation level or kinase activity level of IRAK protein kinase pathway related proteins; and
(b) determining from said level(s) the occurrence of specific neurological disorders, thereby diagnosing neurological disorders in said subject.

More preferably the present invention provides a method for diagnosing Alzheimer's disease.

More preferably the present invention provides a method according to the present invention, wherein said level(s) measured in step (a) are compared to control level(s) or control profile(s), thereby diagnosing neurological disorders in said subject.

Several studies have already indicated that kinases play an important role in different disease mechanisms, including neurological disorders such as Alzheimer's disease. However the studies have studied kinase activity in vitro and the suggested involvement of kinases in Alzheimer's disease has been purely based on expression levels and colocalization studies. For instance, although there is increased expression of glycogen synthase kinase (GSK)-3 in Alzheimer's disease, it is still elusive whether GSK-3 activity is really increased in Alzheimer's disease. Furthermore, the prior art does not disclose the involvement of protein kinases of the IRAK protein kinase family, and preferably IRAK-4 and/or IRAK-1, in neurological disorders such as Alzheimer's disease, Huntington's disease, Parkinson's disease, Creutzfeldt-Jakob disease and other prion diseases. More preferably, protein kinases of the IRAK protein kinase family, and preferably IRAK-4 and/or IRAK-1, have not been associated with Alzheimer's disease.

As referred to in the present application neurological disorders regard disorders that involve the central nervous system (brain, brainstem and cerebellum), the peripheral nervous system (including cranial nerves), and the autonomic nervous system (parts of which are located in both central and peripheral nervous system).

As used in the present invention, the term "sample" refers to a sample obtained from an organism (patient) such as human or animal or from components (e.g., cells) of such an organism. Said sample is preferably obtained from brain tissue from said patient. The sample may therefore also be referred to as "brain tissue sample".

Said sample is preferably a fresh or a fresh frozen sample.

More preferably, said sample refers to a cell lysate of brain tissue obtained through surgical excision, tissue biopsy, fine needle biopsy or core needle biopsy.

Alternatively said sample may be obtained from cerebrospinal fluid (CSF). It should further be noted that the inventors have shown that, notwithstanding the low protein content in CSF, the method of the present invention enables monitoring protein kinase activity in cerebrospinal fluid. It was shown that the protein kinases in CSF are still able to phosphorylate other proteins or peptides.

In a preferred embodiment of the present invention said sample is a sample that has undergone a preparation step prior to the steps according to the method of the present invention. Preferably said preparation step is a step where the protein kinases present in said sample are released from the tissue by lysis. Additionally the kinases in the sample may be stabilized, maintained, enriched or isolated, and the measurement of the kinase activity as performed in step (a) occurs on the enriched or isolated protein kinase sample. By first enriching protein kinases in the sample or isolating protein kinases from the sample the subsequent measurement of the kinase activity will occur in a more efficient and reliable manner. Also the clarity and intensity of the obtained phosphorylation signal will be increased as certain contaminants are being removed during the enriching or isolating step.

The skilled person will appreciate that measuring the level or concentration of IRAK protein kinase(s) in a sample of the subject is performed by means of an immuno assay, mass spectrometry or an activity assay. In particular, the immuno assay may be an ELISA, a Western-blot or cytochemistry, and the activity assay may be based on substrate phosphorylation.

The skilled person will appreciate that measuring the level of IRAK protein kinase phosphorylation, the level of IRAK protein kinase activity and/or the level, phosphorylation level or kinase activity level of IRAK protein kinase pathway related proteins in a sample of the subject is performed using methods commonly known in the art including an immuno assay, mass spectrometry or an activity assay. In particular, the immuno assay may be an ELISA, a Western-blot or cytochemistry, and the activity assay may be based on substrate phosphorylation.

More preferably the method of the present invention measures the kinase activity in said sample, obtained from said subject, thereby obtaining a phosphorylation profile. As used in the present invention, the term "phosphorylation profile" refers to a data set representative for the phosphorylation levels of one or more phosphorylation sites present on each protein kinase substrate. When measuring the kinase activity of a sample by contacting said sample with protein kinase substrates a specific phosphorylation profile is obtained. The phosphorylation profile is generated by the phosphorylation of the protein kinase substrates with the protein kinases present in the sample and it comprises the level of phosphorylation of the phosphorylation sites present on the protein kinase substrates used. A phosphorylation profile can thus be generated when using at least one protein kinase substrate in different test conditions such as for example by comparing the phosphorylation level of a sample on one peptide or protein (protein kinase substrate) in the presence and absence of a protein kinase inhibitor or by comparing the phosphorylation level of a sample on one peptide or protein (protein kinase substrate) with a known phosphorylation level indicative for a neurological disorder. More frequently phosphorylation profiles of a sample will be measured using several protein kinase substrates in the same or sequentially carried out experiments.

It should be noted that a person skilled in the art will appreciate that the methods of the present invention can use phosphorylation profiles as a basis for diagnosing neurological disorders. However, the phosphorylation levels of individual protein kinase substrates can also be used as a basis for diagnosing neurological disorders.

It should be noted that for the measurement of the protein kinase activity, ATP or any other phosphate source needs to be added to the sample when it is contacted with the protein kinase substrates. The presence of ATP will lead to a phosphorylation of the protein kinase substrates. Alternatively, the phosphorylation of the protein kinase substrates can be performed in the absence of exogenous ATP. When no ATP is added during the incubation of the sample with the protein kinase substrates, the endogenous ATP, the ATP naturally present in the sample, will act as the primary source of ATP.

The phosphorylation level of each of the protein kinase substrates can be monitored using any method known in the art. The response of the protein kinase substrates is determined using a detectable signal, said signal resulting from the interaction of the sample with the protein kinase substrates. In determining the interaction of the sample with the protein kinase substrates the signal is either the result of a change in a physical or chemical property of the detectably labelled substrates, or indirectly the result of the interaction of the substrates with a detectably labelled molecule capable of binding to the substrates. For the latter, the molecule that specifically binds to the substrates of interest (e.g., antibody or polynucleotide probe) can be detectably labelled by virtue of containing an atom (e.g., radionuclide), molecule (e.g., fluorescein), or complex that, due to a physical or chemical property, indicates the presence of the molecule. A molecule may also be detectably labelled when it is covalently bound to or otherwise associated with a "reporter" molecule (e.g., a biomolecule such as an enzyme) that acts on a substrate to produce a detectable atom, molecule or other complex.

Detectable labels suitable for use in the present invention include any composition detectable by spectroscopic, photochemical, biochemical, immunochemical, electrical, optical or chemical means. Labels useful in the present invention include biotin for staining with labelled avidin or streptavidin conjugate, magnetic beads (e.g., Dynabeads'), fluorescent dyes (e.g., fluorescein, fluorescein-isothiocyanate (FITC), Texas red, rhodamine, green fluorescent protein and all variation thereof, enhanced green fluorescent protein, yellow fluorescent protein, red fluorescent protein, lissamine, phycoerythrin, Cy2, Cy3, Cy3.5, Cy5, Cy5.5, Cy7, FluorX [Amersham], SYBR Green I & II [Molecular Probes], and the like), radiolabels (e.g., 3H, 1251, 35S, 4C, or 32P), enzymes (e.g., hydrolases, particularly phosphatases such as alkaline phosphatase, esterases and glycosidases, or oxidoreductases, particularly peroxidases such as horse radish peroxidase, and the like), substrates, cofactors, inhibitors, chemilluminescent groups, chromogenic agents, and colorimetric labels such as colloidal gold or coloured glass or plastic (e. g., polystyrene, polypropylene, latex, etc.) beads.

Means of detecting such labels are well known to those of skill in the art. Thus, for example, chemiluminescent and radioactive labels may be detected using photographic film or scintillation counters, and fluorescent markers may be detected using a photodetector to detect emitted light (e.g., as in fluorescence-activated cell sorting). Enzymatic labels are typically detected by providing the enzyme with a substrate and detecting a coloured reaction product produced by the action of the enzyme on the substrate. Colorimetric labels are detected by simply visualizing the coloured label. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the colour associated with the label. Fluorescence resonance energy transfer has been adapted to detect binding of unlabeled ligands, which may be useful on arrays.

In a particular embodiment of the present invention the response of the protein kinase substrates to the sample is determined using detectably labelled antibodies; more in particular fluorescently labelled antibodies. In those embodiments of the invention where the substrates consist of protein kinase substrates, the response of the protein kinase substrates is determined using fluorescently labelled anti-phosphotyrosine antibodies, fluorescently labelled anti-phosphoserine or fluorescently labelled anti-phosphothreonine antibodies. The use of fluorescently labelled anti-phosphotyrosine antibodies or fluorescently labelled anti-phosphoserine or fluorescently labelled anti-phosphothreonine antibodies in the method of the present invention, allows real-time or semi real-time determination of the protein kinase activity and accordingly provides the possibility to express the protein kinase activity as the initial velocity of protein kinase derived from the activity over a certain period of incubation of the sample on the protein kinase substrates.

The inventors have found that measuring kinase activity of a brain tissue sample or CSF, enables diagnosis of patients suffering from neurological disorders, and more preferably Alzheimer's disease. It has been found by monitoring kinase activity in normal brain tissue sample or CSF and brain tissue sample or CSF obtained from patients suffering from Alzheimer's disease, that there is a prominent difference between the kinase activity of both tissue samples or both CSF samples. This difference enables the accurate and diagnosis neurological disorders, and more preferably Alzheimer's disease. Furthermore, a set of peptide markers have shown to provide a good way for the diagnosis of neurological disorders according to the methods of the present invention.

As described in the present invention, the inventors also found the method according to the present invention can be used for predicting the response of a subject, diagnosed with a neurological disorder, on IRAK protein kinase inhibitor(s) or pharmaceutical composition(s) comprising the same.

The present invention therefore relates to a method for predicting the response of a subject, diagnosed with a neurological disorder, on IRAK protein kinase inhibitor(s) or pharmaceutical composition(s) comprising the same comprising the steps of:
(a) measuring in a sample, obtained from said subject, a level of IRAK protein kinase, a level of IRAK protein kinase phosphorylation, a level of IRAK protein kinase activity and/or a level, phosphorylation level or kinase activity level of IRAK protein kinase pathway related proteins; and,
(b) comparing the level(s) or profile(s) determined in step (a) with control value(s) or control profile(s) from which the clinical outcome of the treatment with said IRAK protein kinase inhibitor(s) or said pharmaceutical composition(s) comprising the same is a priori known.

More preferably said method for predicting the response of a subject, diagnosed with a neurological disorder, on IRAK protein kinase inhibitor(s) or pharmaceutical composition(s) comprising the same provides that step (a) is carried out in the presence and in the absence of said IRAK protein kinase inhibitor(s) or said pharmaceutical composition(s) comprising the same and wherein said level(s) or profile(s) in the presence of said IRAK protein kinase inhibitor(s) or said pharmaceutical composition(s) comprising the same is compared to said level(s) or profile(s) in the absence of said IRAK protein kinase inhibitor(s) or said pharmaceutical composition(s) comprising the same thereby determining the response of said patient to said IRAK protein kinase inhibitor(s) or said pharmaceutical composition(s) comprising the same.

Preferably, according to the present invention the methods according to the present invention both for diagnosing and/or predicting the response of a subject to a compound, provide that step (a) provides a phosphorylation profile of said sample, said phosphorylation profile comprising the phosphorylation levels of one or more phosphorylation sites present in any of the peptide markers as listed in Table 1, and wherein from said phosphorylation profile the occurrence of specific neurological disorders is determined, thereby diagnosing neurological disorders in said subject or predicting the response of said subject on IRAK protein kinase inhibitor(s) or pharmaceutical composition(s) comprising the same.

Preferably phosphorylation levels will be studied of, preferably one or more, phosphorylation site(s) present in at least 2, 3, 4, 5, 6, 7, 8, 9 10, 11, 12, 13, 14, 15, 16, 17, 18, 19, 20, 21, 22, 23, 24, 25, 26, 27 or 28 of the peptide markers as listed in Table 1.

More particularly said protein kinase substrates represent the, preferably one or more, phosphorylation sites present in at least 1, 2, 3, 4, 5, 6, 7 or 8 peptide markers with any of Seq.Id.No. 1 to 8. In a more preferred embodiment the protein kinase substrates comprise or consist of, preferably one or more, phosphorylation sites present in all of the peptide markers with any of Seq.Id.No. 1 to 8.

The term "peptide markers" in the context of the present invention refers to the fact that the peptides as listed in Table 1 can be preferably used according to the methods of the present invention to measure the phosphorylation levels of phosphorylation sites of said markers in the presence of protein kinase present in samples. The phosphorylation levels of the individual phosphorylation sites present in said markers may be measured and compared in different ways. Therefore the present invention is not limited to the use of peptides identical to any of these peptide markers as listed in Table 1 as such. The skilled person may easily on the basis of the peptide markers listed in Table 1 design variant peptides compared to the specific peptides in said Table and use such variant peptides in a method for measuring phosphorylation levels of phosphorylation sites common to said peptide markers as listed in Table 1. These variant peptides may have one or more (2, 3, 4, 5, 6, 7, etc.) amino acids more or less than the given peptides and may also have amino acid substitutions (preferably conservative amino acid substitutions) as long as these variant peptides retain at least one or more of the phosphorylation sites of said original peptides as listed in said table. Further the skilled person may also easily carry out the methods according to the present invention by using proteins (full length or N- or C-terminally truncated) comprising the amino acid regions of the "peptide markers" listed in Table 1 as sources for studying the phosphorylation of sites present in the amino acid regions of the peptides listed in Table 1. Also the skilled person may use peptide mimetics.

The protein kinase substrates as used in the methods described herein, are meant to include peptides, proteins or peptide mimetics comprising one or more of the phosphorylation sites of the peptide markers of Table 1. Said one or more phosphorylation sites are specifically phosphorylated by the protein kinases present in the sample thereby providing a phosphorylation profile. More preferably the protein kinase substrates (peptides, proteins or peptide mimetics) as used in the method of the present invention comprise one or more of the phosphorylation sites present in at least two peptide markers as listed in Table 1. More particularly said protein kinase substrates represent the one or more phosphorylation sites present in at least 2, 3, 4, 5, 6, 7, 8, 9 10, 11, 12, 13, 14, 15, 16, 17, 18, 19, 20, 21, 22, 23, 24, 25, 26, 27 or 28 of the peptide markers as listed in Table 1. In a more preferred embodiment the protein kinase substrates comprises or consists of one or more phosphorylation sites present in all of the peptide markers listed in Table 1.

More particularly said protein kinase substrates represent the, preferably one or more, phosphorylation sites present in at least 1, 2, 3, 4, 5, 6, 7 or 8 peptide markers with any of Seq.Id.No. 1 to 8. In a more preferred embodiment the protein kinase substrates comprise or consist of, preferably one or more, phosphorylation sites present in all of the peptide markers with any of Seq.Id.No. 1 to 8.

A person skilled in the art will appreciate that the phosphorylation sites present in a single peptide marker as listed in Table 1 enable determining the diagnosis of neurological disorders, preferably Alzheimer's disease. The peptide marker as listed in Table 1 also enables the diagnosis of neurological disorders closely related to Alzheimer's disease. Especially neurological disorders from the group comprising Alzheimer's disease, Huntington's disease, Parkinson's disease, Creutzfeldt-Jakob disease, other prion diseases and multiple sclerosis. However, when the number of peptide markers as listed in Table 1 increases, so will increase the specificity and sensitivity of the method according to the present invention. When for example only one protein kinase substrate comprising the phosphorylation sites of a single peptide marker as listed in table 1 is used for diagnostical of response predictive purposes the accuracy of the method will be lower, compared to a method where the diagnosis or response prediction uses multiple protein kinase substrates comprising the phosphorylation sites of multiple peptide markers as listed in table 1. The highest method accuracy will be obtained when all protein kinase substrates comprising the phosphorylation sites of all peptide markers as listed in table 1 are used.

**Table 1: list of 28 peptide markers comprising phosphorylation sites used for determining the IRAK kinase activity, their sequence and Seq.Id.No.**

| Seq.Id.No | Peptide marker Name | Peptide marker Sequence |
|---|---|---|
| 1 | ACM4_456_468_T459/T463 | CNATFKKTFRHLL |
| 2 | ACM5_494_506_T501/T505/Y495 | CYALCN RTFRKTF |
| 3 | ACM5_498_510_T501/T505 | CN RTFRKTFKM LL |
| 4 | FIBA_569_581_S572/S576/S577/S578/S580/Y579 | EFPSRGKSSSYSK |
| 5 | KCC2G_278_289_S280/T287 | VASMMHRQETVE |
| 6 | MARCS_159_171_S162/S166/S169 | FKKSFKLSGFSFK |
| 7 | P2AB_297_309_T304/Y307 | EPHVTRRTPDYFL |
| 8 | RADI_559_569_Y562/T564 | RDKYKTLRQIR |
| 9 | RB_242_254_T252/S249 | AVIPINGSPRTPR |
| 10 | LMNA_192_204_T199 | DAENRLQTMKEEL |
| 11 | BCKD_45_57_T49/T51/S47/S52/S57/Y54 | ERSKTVTSFYNQS |
| 12 | NEK2_171_184_T174/T178/S183/Y180/Y181 | FAKTFVGTPYYMS |
| 13 | PTN12_32_44_T40/T44/S39/Y42 | FMRLRRLSTKYRT |
| 14 | LAM1_15_27_T18/T19/T24/S22/S27 | GGPTTPLSPTRLS |
| 15 | CDK7_163_175_T170/T175/S164/Y169 | GSPNRAYTHQVVT |
| 16 | ADDB_696_708_S697/S699/S701/S703 | GSPSKSPSKKKKK |
| 17 | DCX_49_61_T56/S57 | HFDERDKTSRNMR |
| 18 | RS6 228 240 S235/S236/S240 | IAKRRRLSSLRAS |
| 19 | ACM1_444_456_T455/S451 | KIPKRPGSVHRTP |
| 20 | ADDB_706_718_T711/S713/S718 | KKKFRTPSFLKKS |
| 21 | MARCS_151_163_S158/S162 | KKKKKRFSFKKSF |
| 22 | INSR_1368_1380_T1375/S1379 | KKN GRI LTLPRSN |
| 23 | RAP1B_172_184_S179/S180 | PGKARKKSSCQLL |
| 24 | MP2K1_286_298_T291/S297/S298 | PPRPRTPGRPLSS |
| 25 | FOXO3_25_37_T32/S26/S30 | QSRPRSCTWPLQR |
| 26 | KPCB_18_30_S24_A24S | RFARKGSLRQKNV |
| 27 | RAF1_252_264_T258/T260/S252/S257/S259 | SQRQRSTSTPNVH |
| 28 | ATF2_47-59_T51/T53/T55 | VADOTPTPTRFLK |

It should further be noted that according to a preferred embodiment of the present invention the peptide markers as listed in Table 1 can be used as such for carrying out the methods according to the present invention. The present invention however also includes the use of analogs and combinations of these peptide markers for use in the method according to the present invention. The peptide marker analogs include peptide markers which show a sequence identity of more than 70%, preferably more than 80% and more preferably more than 90%.

In yet another embodiment, the present invention relates to a method according to the present invention wherein said kinase activity of said brain tissue sample or CSF from said subject is measured in the presence and in the absence of a protein kinase inhibitor, thereby providing a phosphorylation profile of said sample in the presence and in the absence of a protein kinase inhibitor; and, determining from said phosphorylation profiles in the presence and in the absence of a protein kinase inhibitor the differential phosphorylation level, said differential phosphorylation level indicating the diagnosis of neurological disorders, preferably Alzheimer's disease in said subject.

The inventors have further found that by comparing phosphorylation profiles in the presence and in the absence of a protein kinase inhibitor the difference between experimental variation can be reduced.

The term "differential phosphorylation level" as used herein therefore refers to a data set comprising comparison data form the phosphorylation profiles in the presence and in the absence of a protein kinase inhibitor. The statistical analysis of the differential phosphorylation level can be done using multivariate and/or univariate statistical methods known in the art. The differential phosphorylation levels are obtained by (numerically) comparing the peptide phosphorylation levels or profiles in the presence and in the absence of the protein kinase inhibitor in the same sample, for instance, but not limited to, providing ratios or differences of the profiles obtained in the presence and the absence of the protein kinase inhibitor.

In addition, because the differential phosphorylation level is generated by comparing the phosphorylation levels or profiles of the same sample in the presence and the absence of the protein kinase inhibitor, preferably during a parallel series of measurements run in the same instrument, the differential phosphorylation level is surprisingly found to be less affected by variation, for example biological variation, experimental variation, compared to single phosphorylation levels or profiles. This provides a more robust, more sensitive, more reproducible and more reliable method for diagnosing neurological disorders, preferably Alzheimer's disease.

According to another embodiment, the present invention relates to a method according to the present invention wherein additionally a classifier parameter is established from said phosphorylation profile(s) of said sample, said classifier parameter determining the diagnosis of neurological disorders, preferably Alzheimer's disease, in said subject.

By establishing a classifier parameter for diagnosing or response prediction of neurological disorders, preferably Alzheimer's disease, the method of the present invention establishes a criterion for analysing the results obtained from the method of the present invention. This criterion enables a person to provide a diagnosis or response prediction on the basis of a single or limited number of data. The person providing the diagnosis or response prediction does not have to interpret an entire set of data, but rather bases his conclusion on the basis of a single or limited number of criteria.

The term "classifier parameter" as used herein represents a discriminating value which has been determined by establishing a single phosphorylation profile or a phosphorylation profile in the presence and in the absence of a protein kinase inhibitor.

Said discriminating value can be used for the diagnosis or response prediction of neurological disorders, preferably Alzheimer's disease. The classifier parameter includes information regarding the phosphorylation level of several protein kinase substrates. Classification is a procedure in which individual items are placed into groups based on quantitative information on one or more characteristics inherent in the items (e.g. phosphorylation levels or profiles of a sample) and based on a training set of previously labelled items (clinical response to a pharmacotherapy). The classifier parameter is calculated by applying a "classifier" to the measured phosphorylation levels of a sample. Based on the classifying parameter a sample is assigned to (or predicted to belong to) a class (determining the diagnosis or response prediction of neurological disorders, preferably Alzheimer's disease). The classifier has been previously determined by comparing samples which are known to belong to the respective relevant classes. For instance the classifier may be a mathematical function that uses information regarding the phosphorylation level of several protein kinase substrates which individual protein kinase substrates can be weighted based on the measured phosphorylation level of a number of protein kinase substrates (or values derived from that). Several methods are known in the art for developing a classifier including the neural network (Multi-layer Perceptron), support vector machines, k-nearest neighbours, Gaussian mixture model, naive bayes, decision tree, RBF classifiers, random forest, disciminant analysis, linear discriminant analysis, quadratic discriminant analysis, discriminant analysis - principal component analysis, partial least squares discriminant analysis, generalized distance regression and elastic net classification.

It is not relevant to give an exact threshold value for the classifier parameter. A relevant threshold value can be obtained by correlating the sensitivity and specificity and the sensitivity/specificity for any threshold value. A threshold value resulting in a high sensitivity results in a lower specificity and vice versa. If one wants to diagnose the neurological disorders, preferably Alzheimer's disease, with a high certainty, then the specificity will be lower and some false positive results will be included.

It is thus up to the diagnostic engineers to determine which level of sensitivity/specificity is desirable and how much loss in specificity is tolerable. The chosen threshold level could be dependent on other diagnostic parameters used in combination with the present method by the diagnostic engineers.

In yet another embodiment, the present invention relates to a method according to the present invention wherein said classifier parameter indicates the presence of a neurological disorder, preferably Alzheimer's disease, in said subject if said classifier parameter is above a first predetermined threshold level, and wherein said classifier parameter indicates the absence of a neurological disorder, preferably Alzheimer's disease, in said subject if said classifier parameter is below a second predetermined threshold level.

According to another embodiment, the present invention relates to the method of the present invention wherein said phosphorylation profile, differential phosphorylation profile or level or said classifier parameter indicates the presence, absence or undetermined or no-diagnosis of a neurological disorder, preferably Alzheimer's disease, in said subject.

In yet another embodiment, the present invention relates to a diagnostical method according to the present invention wherein step (b) is replaced by steps (c) and (d) as provided below. The method according to the present invention may therefore comprise the steps of:
(a) measuring the IRAK protein kinase activity of a sample from said subject, thereby providing the phosphorylation profile of said sample; and,
(c) comparing said phosphorylation profile to a first and a second reference phosphorylation profile; said first reference phosphorylation profile being representative for a neurological disorder, preferably Alzheimer's disease, positive subject and said second reference phosphorylation profile being representative for a neurological disorder, preferably Alzheimer's disease, negative subject; and,
(d) determining the neurological disorder, preferably Alzheimer's disease, status on the basis of the comparison of said phosphorylation profile with said first and said second reference phosphorylation profile.

As used herein, a "reference phosphorylation profile" refers to a profile obtained through measuring the phosphorylation levels of protein kinase substrates. More specifically, a neurological disorder, preferably Alzheimer's disease, positive reference phosphorylation profile as used herein, refers to a reference phosphorylation profile wherein the phosphorylation levels of a set of protein kinase substrates are representative for a neurological disorder, preferably Alzheimer's disease, positive subject. Additionally, a neurological disorder, preferably Alzheimer's disease, negative reference phosphorylation profile as used herein, refers to a reference phosphorylation profile wherein the phosphorylation levels of a set of protein kinase substrates are representative for a neurological disorder, preferably Alzheimer's disease, negative subject.

The neurological disorder, preferably Alzheimer's disease, can further be defined as the error-weighted log ratio average of the phosphorylation difference for the group of protein kinase substrates able to determine the neurological disorder, preferably Alzheimer's disease, status of a subject.

As used in the present application the diagnosis of a neurological disorder, preferably Alzheimer's disease is generally divided into two types of diagnosis, said subject is either indicated as an Alzheimer's disease positive subject, an Alzheimer's disease negative subject or an undetermined subject.

Another embodiment of the present invention relates to a method according to the present invention for diagnosis and/or response prediction wherein said kinase substrates carrying phosphorylation sites are located or immobilized on a solid support, and preferably a porous solid support. Preferably said immobilized kinase substrates carrying phosphorylation sites will be immobilized proteins, peptides or peptide mimetics. In a preferred embodiment of the present invention peptides are immobilized on a solid support.

As used herein "peptide" refers to a short truncated protein generally consisting of 2 to 100, preferably 2 to 30, more preferably 5 to 30 and even more preferably 13 to 18 naturally occurring or synthetic amino acids which can also be further modified including covalently linking the peptide bonds of the alpha carboxyl group of a first amino acid and the alpha amino group of a second amino acid by eliminating a molecule of water. The amino acids can be either those naturally occurring amino acids or chemically synthesized variants of such amino acids or modified forms of these amino acids which can be altered from their basic chemical structure by addition of other chemical groups which can be found to be covalently attached to them in naturally occurring compounds.

As used herein "protein" refers to a polypeptide made of amino acids arranged in a linear chain and joined together by peptide bonds between the carboxyl and amino groups of adjacent amino acid residues.

As used herein "peptide mimetics" refers to organic compounds which are structurally similar to peptides and similar to the peptide sequences list in Table 1. The peptide mimetics are typically designed from existing peptides to alter the molecules characteristics. Improved characteristics can involve, for example improved stability such as resistance to enzymatic degradation, or enhanced biological activity, improved affinity by restricted preferred conformations and ease of synthesis. Structural modifications in the peptidomimetic in comparison to a peptide, can involve backbone modifications as well as side chain modification.

For measuring the kinase activity of the sample a large variety of methods and formats are known in the art. The kinase activity can for example be measured using ELISA and multiplex ELISA techniques, blotting methods, mass spectrometry, capillary electrophoresis, bead arrays, macroarrays, microarrays or any other method known in the art. Depending on the type of kinase activity measurement method the solid support on which the proteins, peptides or peptide mimetics are fixed may vary. Whereas in ELISA the protein kinase substrates are attached to the surface of the microtiterplates, in microarrays the protein kinase substrates are immobilized on and/or in the microarray substrate.

In a preferred embodiment of the present invention the protein kinase substrates are immobilized on an array, and preferably a microarray of protein kinase substrates wherein the protein kinase substrates are immobilized onto a solid support or another carrier. The immobilization can be either the attachment or adherence of two or more protein kinase substrate molecules to the surface of the carrier including attachment or adherence to the inner surface of said carrier in the case of e.g. a porous or flow-through solid support.

In a preferred embodiment of the present invention, the array of protein kinase substrates is a flow-through array. The flow-through array as used herein could be made of any carrier material having oriented through-going channels as are generally known in the art, such as for example described in PCT patent publication WO 01/19517. Typically the carrier is made from a metal oxide, glass, silicon oxide or cellulose. In a particular embodiment the carrier material is made of a metal oxide selected from the group consisting of zinc oxide, zirconium oxide, tin oxide, aluminium oxide, titanium oxide and thallium; in a more particular embodiment the metal oxide consists of aluminium oxide.

Accordingly, in a further embodiment of the present invention said array is a Pamchip®.

More preferably said peptide markers are peptide markers for IRAK protein kinases and preferably IRAK-1 and/or IRAK-4 protein kinases.

In a further embodiment, the present invention relates to a method according to the present invention wherein said solid support (microarray) comprises each of the peptide as listed in Table 1 immobilized thereto.

The present invention also relates according another embodiment to an array for carrying out the methods of the present invention, said array comprising immobilized proteins, peptides or peptide mimetics comprising one or more phosphorylation sites present in any of the peptide markers as listed in table 1. Said proteins, peptides or peptide mimetics are preferably at least 25% of proteins, peptides or peptide mimetics on said array.

More particularly said array comprises immobilized proteins, peptides or peptide mimetics comprising one or more phosphorylation sites as described in detail above representing the peptide markers as listed in table 1. Additionally said proteins, peptides or peptide mimetics are preferably at least 25%, at least 50%, at least 70%, at least 80%, at least 90% or 100% of the proteins, peptides or peptide mimetics on said array.

The types of arrays to be used according to this embodiment are known in the art and are further detailed above.

The present invention also relates in another embodiment to a computer program product for use in conjunction with a computer having a processor and a memory connected to the processor, said computer program product comprising a computer readable storage medium having a computer program mechanism encoded thereon, wherein said computer program mechanism may be loaded into the memory of said computer and cause said computer to carry out a method according to the present invention.

The present invention further relates to a computer system comprising a processor, and a memory coupled to said processor and encoding one or more programs, wherein said one or more programs instruct the processor to carry out a method according to the present invention.

The present invention also relates in another embodiment to a kit for diagnosing neurological disorders, preferably Alzheimer's disease, comprising at least one array according to the present invention, and optionally a computer readable medium having recorded thereon one or more programs for carrying out the method according to the present invention.

Since the present inventors have identified a surprisingly useful set of peptide markers to be used in methods for diagnosing neurological disorders, preferably Alzheimer's disease, the skilled man may carry out any method as defined above wherein he measures the kinase activity of any of the peptide markers of Table 1. Also this method may be carried out using the amount and type of peptides, proteins or protein mimetics as defined above. The formats for carrying out this methods are also as for the methods described above.

### EXAMPLES

### EXAMPLE 1 - Example showing the involvement of protein kinases of the IRAK family in Alzheimer's disease

A 10 µl aliquot of freshly frozen post mortem cerebrospinal fluid was added to a kinase incubation mixture containing ATP and detection antibodies and placed on a PamChip® STK array that was blocked with 2% BSA. After loading of the reaction mixtures onto Pamchip arrays comprising 140 substrates for protein kinases, incubation was commenced thereby measuring the kinase activity of the sample. As controls, arrays were incubated without ATP or without CSF. After 60 cycles of pumping the incubation mixture through the array, the mixture was removed and the array washed three times with PBS. A mixture of detection antibodies was added to the array and pumped up and down. Images were made at intervals of ten cycles of pumping. After 60 cycles of incubation and imaging, the detection mixture was removed and the array was washed with PBS. Images were collected at different exposure times. Signals for each spot on the image were quantified. The signals intensities were used for further analysis. The signals obtained in the incubations without ATP were subtracted from the signals obtained in the presence of ATP. Table 2 illustrates the differences in phosphorylation of peptide markers with Seq. Id. Nos. 1 to 8 in samples from non-demented (CSF 1-3) and demented patients (CSF 4-6).

**Table 2**

| | **CSF1** | **CSF2** | **CSF3** | **CSF4** | **CSF5** | **CSF6** |
|---|---|---|---|---|---|---|
| | Non-demented control, br 0,O | | | Alzheimer's disease, br. 6,C | | |
| Seq. Id. No. 1 | -134 | -77 | 62 | 663 | 366 | 548 |
| Seq. Id. No. 2 | -123 | 457 | 546 | 1415 | 1293 | 1361 |
| Seq. Id. No. 3 | 56 | 228 | 85 | 1291 | 920 | 664 |
| Seq. Id. No. 4 | 19 | 9 | 7 | 552 | 533 | 503 |
| Seq. Id. No. 5 | -882 | -124 | -398 | 466 | 327 | 898 |
| Seq. Id. No. 6 | -220 | -67 | 78 | 661 | 580 | 404 |
| Seq. Id. No. 7 | 116 | 222 | 134 | 268 | 417 | 515 |
| Seq. Id. No. 8 | 882 | 2856 | 633 | 4771 | 4429 | 3756 |

The measurements of the kinase activity of post mortem brain tissue samples obtained from Alzheimer patients and non-demented cases indicated that the phosphorylation profiles on a PamChip STK array were clearly different. More specifically, the difference in kinase activity was found to be associated with protein kinases of the IRAK family. This conclusion was confirmed by comparing the phosphorylation profiles of these samples and with phosphorylation profiles generated by recombinant IRAK-4 kinase. The presence of IRAK-4 in post mortem brain tissue has further been confirmed by Western blot analyses using commercially available antibodies.

### EXAMPLE 2 - Additional example confirming the involvement of protein kinases of the IRAK family in Alzheimer's disease

The IRAK-4 expression in Alzheimer's disease was further validated by immunohistochemistry using post mortem brain tissue derived from 22 different cases, 11 cases of Alzheimer's disease and 11 non-demented control cases. Immunohistochemical staining shows localization of IRAK-4 in astrocytes and microglia. These results were obtained using different commercially available antibodies.

The IRAK-4 expression in astrocytes was further validated in vitro. Western blot analysis and immunocytochemistry shows presence of IRAK-4 in U373 cells (astrocytoma cell line) and primary human adult astrocytes obtained after surgery or isolated from post mortem derived brain tissue. Staining for the presence of IRAK-4 on Western blot and immunocytochemistry was performed using commercially available antibodies.

### EXAMPLE 3 - The use of IRAK protein kinase inhibitor for the treatment of Alzheimer's Disease

IL-1β has been implicated in both the initiation and propagation of neuroinflammatory changes seen in Alzheimer's disease patients. One of the main mechanisms of IL-1β is the induction of interleukin 6 (IL-6) secretion, which propagates the neuroinflammatory response in Alzheimer's disease patients. Using primary human adult astrocytes this inflammatory response as well as modulators (i.e. potential drugs for therapy) can be studied in vitro. Figure 1 shows the secretion of IL-6 by U373 astrocytoma cells and human primary astrocytes after 6 hours in culture. In the presence of IL-1β (10 U/ml) these cells secrete IL-6 in the culture supernatant, which can be measured by ELISA. The presence of IRAK1/4 inhibitor I dose-dependently inhibits IL-6 secretion. Cells were incubated with or without IL-1β (10 U/ml) for 6 hours in the presence of different concentration of IRAK1/4 inhibitor I (1250, 2500, or 5000 nM). IL-6 levels (pg/ml) in the culture supernatants were determined by ELISA. Shown are mean levels +/- S.D. of three replicate stimulations.

This indicates that inhibition of IRAK1/4 could reduce the neuroinflammatory response in Alzheimer patients.

## Claims

1. Inhibitors of the IRAK protein kinase family for use in the treatment of neurological disorders such as Alzheimer's disease, Huntington's disease, Parkinson's disease, Creutzfeldt-Jakob disease, other prion diseases and multiple sclerosis.

2. IRAK protein kinase inhibitors for use as in Claim 1, wherein said neurological disorder is Alzheimer's disease.

3. IRAK protein kinase inhibitors for use as in Claims 1 or 2, wherein said inhibitors of the IRAK protein kinase family are IRAK-4 or IRAK-1 protein kinase inhibitors.

4. IRAK protein kinase inhibitors for use as in any of Claims 1 to 3, wherein said inhibitors of the IRAK protein kinase family are selected from the group consisting of small molecules, aptamers and/or genetic interferers directed toward the IRAK protein kinase family.

5. A pharmaceutical composition comprising inhibitors of the IRAK protein kinase family, and preferably IRAK-4 or IRAK-1 protein kinase inhibitors, for use in the treatment of neurological disorders such as Alzheimer's disease, Huntington's disease, Parkinson's disease, Creutzfeldt-Jakob disease, other prion diseases and multiple sclerosis and preferably Alzheimer's disease.

6. The pharmaceutical composition according to claim 5, wherein said inhibitors of the IRAK protein kinase family are selected from the group consisting of small molecules, aptamers and/or genetic interferers directed toward the IRAK protein kinase family.

7. A method for diagnosing neurological disorders such as Alzheimer's disease, Huntington's disease, Parkinson's disease, Creutzfeldt-Jakob disease, other prion diseases and multiple sclerosis, in a subject, comprising the steps of:
(a) measuring in a sample, obtained from said subject, a level of IRAK protein kinase, a level of IRAK protein kinase phosphorylation, a level of IRAK protein kinase activity and/or a level, phosphorylation level or kinase activity level of IRAK protein kinase pathway related proteins; and
(b) determining from said level(s) the occurrence of specific neurological disorders, thereby diagnosing neurological disorders in said subject.

8. Method according to Claim 7, wherein said level(s) measured in step (a) are compared to control level(s) or control profile(s), thereby diagnosing neurological disorders in said subject.

9. A method for predicting the response of a subject, diagnosed with a neurological disorder, on IRAK protein kinase inhibitor(s) or pharmaceutical composition(s) comprising the same, comprising the steps of:
(a) measuring in a sample, obtained from said subject, a level of IRAK protein kinase, a level of IRAK protein kinase phosphorylation, a level of IRAK protein kinase activity and/or a level, phosphorylation level or kinase activity level of IRAK protein kinase pathway related proteins; and,
(b) comparing the level(s) or profile(s) determined in step (a) with control value(s) or control profile(s) from which the clinical outcome of the treatment with said IRAK protein kinase inhibitor(s) or said pharmaceutical composition(s) comprising the same is a priori known.

10. Method according to Claim 9, wherein the measurement of step (a) is carried out in the presence and in the absence of said IRAK protein kinase inhibitor(s) or said pharmaceutical composition(s) comprising the same and wherein said level(s) or profile(s) in the presence of said IRAK protein kinase inhibitor(s) or said pharmaceutical composition(s) comprising the same is compared to said level(s) or profile(s) in the absence of said IRAK protein kinase inhibitor(s) or said pharmaceutical composition(s) comprising the same thereby determining the response of said patient to said IRAK protein kinase inhibitor(s) or said pharmaceutical composition(s) comprising the same.

11. Method according to any of Claims 7 to 10, wherein the measurement of step (a) provides a phosphorylation profile of said sample, said phosphorylation profile comprising the phosphorylation levels of one or more phosphorylation sites present in any of the peptide markers as listed in Table 1, and wherein from said phosphorylation profile the occurrence of specific neurological disorders is determined, thereby diagnosing neurological disorders in said subject or predicting the response of said subject on IRAK protein kinase inhibitor(s) or pharmaceutical composition(s) comprising the same.

12. The method according to Claim 11, wherein said peptide markers are peptide markers for IRAK protein kinases and preferably IRAK-1 and/or IRAK-4 protein kinases.

13. The method according to claims 11 or 12, wherein said phosphorylation sites are present on proteins, peptides or peptide mimetics immobilized on a solid support, and preferably a porous solid support.

14. A method for identifying drug compounds relevant to neurological disorders, comprising the steps of:
(a) measuring, a level of IRAK protein kinase, a level of IRAK protein kinase phosphorylation, a level of IRAK protein kinase activity and/or a level, phosphorylation level or kinase activity level of IRAK protein kinase pathway related proteins in a sample, obtained from a subject suffering from said neurological disorder, in the presence and in the absence of a drug compound; and,
(b) comparing the level(s) or profile(s) determined in step (a) in the presence of said drug compound with the level(s) or profile(s) determined in step (a) in the absence of said drug compound, thereby determining whether said drug compound is relevant for said neurological disorder.

15. An array for carrying out the method of any of Claims 7 to 14, said array comprising immobilized proteins, peptides or peptide mimetics comprising phosphorylation sites present in at least two peptide markers as listed in Table 1.
